(19) Europäisches Patentamt / European Patent Office / Office européen des brevets

(11) **EP 4 407 559 A1**

(12) **EUROPEAN PATENT APPLICATION**
published in accordance with Art. 153(4) EPC

(43) Date of publication:
**31.07.2024 Bulletin 2024/31**

(51) International Patent Classification (IPC):
**G06T 11/00** $^{(2006.01)}$

(21) Application number: **22872036.3**

(86) International application number:
**PCT/CN2022/120404**

(22) Date of filing: **22.09.2022**

(87) International publication number:
**WO 2023/045991 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.09.2021 CN 202111109543**

(71) Applicant: **Towardpi (Beijing) Medical Technology Ltd.**
**Beijing 102206 (CN)**

(72) Inventors:
• **YANG, Zhi**
  **Beijing 102206 (CN)**
• **WANG, Xiao**
  **Beijing 102206 (CN)**

(74) Representative: **Murgitroyd & Company**
**165-169 Scotland Street**
**Glasgow G5 8PL (GB)**

(54) **OPTICAL COHERENCE TOMOGRAPHY ANGIOGRAPHY METHOD AND APPARATUS, AND ELECTRONIC DEVICE AND STORAGE MEDIUM**

(57) The present disclosure provides a method and an apparatus for optical coherence tomography angiography, and an electronic device and a storage medium. The method includes: acquiring time domain signals of a target area, the time domain signals being N interference spectrum signals obtained by repeating A-scans on the target area N times; performing scale transform on respective interference spectrum signals in the N interference spectrum signals based on k scales, to obtain N × k scale-transformed signals; performing Fourier transform on each of the scale-transformed signals and taking a logarithm, to obtain an axial frequency domain signal in a logarithmic space, and denoising the axial frequency domain signal; dividing, based on the k scales, N × k of the axial frequency domain signals denoised into k sets of single-scale signals, and performing decorrelation calculation on each set of the single-scale signals to obtain single-scale blood flow signals; obtaining a multi-scale blood flow signal based on k sets of the single-scale blood flow signals; and obtaining a blood flow image based on the multi-scale blood flow signal. The present disclosure can improve an accuracy of identifying a blood flow signal.

FIG. 1

**Description**

**TECHNICAL FIELD**

The present disclosure relates to the field of optical coherence tomography, and specifically to a method and an apparatus for optical coherence tomography angiography, and an electronic device and a storage medium.

**BACKGROUND**

Optical Coherence Tomography (OCT) is a non-invasive imaging approach with a high sensitivity, a high resolution and a high speed, which has been widely used in diagnosis of fundus diseases, which is of significance for detection and treatment of ophthalmic diseases.

In OCT as a tomographic imaging approach, the fundus is scanned and imaged using coherence of light, where each scan is called one A-scan and a combination of multiple consecutive scans is called one B-scan, which is also the commonly seen cross sectional view of the OCT and is the most important imaging approach of OCT in medical diagnosis.

In the field of OCT, OCT Angiography (OCTA) is a new technology developed in recent years. An OCTA image for retina can intuitively display the pattern and the distribution of the blood flow in the retina, and the like, which plays an important role in diagnosis of fundus diseases. In a stationary eyeball, blood cells in blood vessels are the only moving thing in the fundus. Therefore, for OCTA, the red blood cells flowing in the blood vessels can be used as a contrast agent to measure the OCT signal difference caused by moving cells, provide blood flow information, and reconstruct a three-dimensional structure of the retina blood vessels and choroidal blood vessels quickly and non-invasively, to thus implement visualization of the vascular network. During angiography, it is necessary to repeatedly photograph the same position multiple times, and calculate the differences among the B-scans photographed multiple times to obtain the blood flow signal at the current position.

Nevertheless, the high axial resolution of OCTA makes it very sensitive to pulsation and jitter noise, easily leading to imaging errors. As such, an approach of reducing the axial resolution is generally used to reduce the impact of noise; however, reducing the axial resolution may be accompanied by a reduced accuracy of identifying blood flow signals.

**SUMMARY**

In order to improve the accuracy of identifying a blood flow signal, the present disclosure provides a method and an apparatus for optical coherence tomography angiography, and an electronic device and a storage medium.

In a first aspect, the present disclosure provides a method for optical coherence tomography angiography, specifically as follows:

A method for optical coherence tomography angiography, comprising:

acquiring time domain signals of a target area, the time domain signals being N interference spectrum signals obtained by repeating A-scans on the target area N times;

performing scale transform on respective interference spectrum signals in the N interference spectrum signals based on k scales, to obtain N × k scale-transformed signals;

wherein performing scale transform on any interference spectrum signal based on the k scales comprises: decomposing the any interference spectrum signal based on each of the k scales, to obtain k scale-transformed signals corresponding to the any interference spectrum signal;

performing Fourier transform on each of the scale-transformed signals and taking a logarithm, to obtain an axial frequency domain signal in a logarithmic space, and denoising the axial frequency domain signal;

dividing, based on the k scales, N × k of the axial frequency domain signals denoised into k sets of single-scale signals, and performing decorrelation calculation on each set of the single-scale signals to obtain single-scale blood flow signals;

obtaining a multi-scale blood flow signal based on k sets of the single-scale blood flow signals; and

obtaining a blood flow image based on the multi-scale blood flow signal.

According to the above technical solution, the blood flow signal is a non-stationary signal, and its changes are localized. An interference spectrum signal is decomposed into multiple segments after a scale transform. In the subsequent decorrelation calculation, axial frequency domain signals corresponding to each segment of the interference spectrum signal corresponding to the same target area at different times can be compared. Through segmented comparison, changing signals in a non-stationary state can be captured more accurately, to thus implement accurate extraction of blood flow signals.

Moreover, multi-scale transform is performed on an interference spectrum signal, and the amount of decomposed segments corresponding to each scale is different, i.e., the scales of segmented comparison are different, such that the changes of non-stationary signals can be measured at different scales. Therefore, simultaneously performing scale transform and fusion of multiple scales on an interference spectrum signal can reduce the misjudgment rate of a single scale while improving the accuracy and processing efficiency of blood flow signal extraction; and as all the points in the interference spectrum signal are reserved, more details of the image can be restored, thus improving the signal-to-noise ratio.

In a possible implementation, when the interference spectrum signals are corresponded to a sampling time period, decomposing the any interference spectrum signal based on each of the k scales, to obtain the k scale-transformed signals corresponding to the any interference spectrum signal, comprises:
decomposing the any interference spectrum signal based on any scale in the k scales, to obtain scale-transformed signals of the any scale corresponding to the any interference spectrum signal, comprising:

determining a decomposed signal corresponding to the any scale, the decomposed signal being a signal within the sampling time period T, the decomposed signal comprising at least one time set that includes a first time period and a second time period, two of the first time periods adjacent to each other, or two of the second time periods adjacent to each other;

multiplying the any interference spectrum signal corresponding to the first time period by a forward signal to obtain a first signal;

multiplying the any interference spectrum signal corresponding to the second time period by a reverse signal to obtain a second signal; and obtaining, based on at least one of the first signal and at least one of the second signal, scale-transformed signals of the any interference spectrum signal corresponding to the any scale.

With the above technical solution, the sampling time period T is divided based on at least one time set corresponding to the scale, and the interference spectrum signal is segmented into at least one first signal and at least one second signal different from each other based on a forward signal and a reverse signal, to implement segmentation of the interference spectrum signal and thus obtain scale-transformed signals corresponding to the scale.

In a possible implementation, denoising the axial frequency domain signal within the logarithmic space, to obtain the axial frequency domain signal denoised, comprises:
determining a segmentation threshold;

if an amplitude of the axial frequency domain signal is greater than or equal to the segmentation threshold, the axial frequency domain signal denoised being equal to the axial frequency domain signal within the logarithmic space; and

if the amplitude of the axial frequency domain signal is less than the segmentation threshold, the axial frequency domain signal denoised being equal to the segmentation threshold.

With the above technical solution, noise reduction and segmentation are performed on axial frequency domain signals based on the segmentation threshold, to improve the signal-to-noise ratio.

In a possible implementation, the determining the segmentation threshold comprises:

extracting, based on a clustering algorithm, noise signals corresponding to all of the axial frequency domain signals; and

determining the segmentation threshold based on the noise signals.

With the above technical solution, noise signals are extracted and the segmentation threshold is determined through unsupervised learning, and the threshold is determined automatically through axial frequency domain signals, to improve the accuracy of determining the segmentation threshold and increase the signal-to-noise ratio.

In a possible implementation, the performing decorrelation calculation on each set of the single-scale signals to obtain the single-scale blood flow signals, comprising:

determining first-order statistics, second-order statistics, and third-order statistics of each set of the single-scale signals; and

performing the decorrelation calculation based on the first-order statistics, the second-order statistics, and the third-order statistics to determine the single-scale blood flow signals.

With the above technical solution, more useful information in single-scale signals can be extracted by using high-order statistics, and decorrelation calculation is performed, to improve the accuracy of extracting single-scale blood flow signals.

In a possible implementation, after performing the Fourier transform on each of the scale-transformed signals and taking the logarithm, to obtain the axial frequency domain signal in the logarithmic space, it further comprises:

performing, based on a phase correlation algorithm, an alignment operation on all of the axial frequency domain signals;

wherein the denoised axial frequency domain signals are the signals obtained by denoising the axial frequency domain signals after the alignment operation

With the above technical solution, the alignment operation can reduce the interference of jitter to the axial frequency domain signals, thereby achieving the effect of increasing the signal-to-noise ratio.

In a possible implementation, prior to the performing scale transform on the respective interference spectrum signals in the N interference spectrum signals based on the k scales, the method further comprises: performing dispersion compensation on the respective interference spectrum signals in the time domain signals.

In a second aspect, the present disclosure provides an apparatus for optical coherence tomography angiography, specifically as follows:

An apparatus for optical coherence tomography angiography, comprising:

an acquiring module configured to acquire time domain signals of a target area, the time domain signals being N interference spectrum signals obtained by repeating A-scans on the target area N times;

a scale-transforming module configured to perform scale transform on respective interference spectrum signals in the N interference spectrum signals based on k scales, to obtain N $\times$ k scale-transformed signals;

wherein performing scale transform on any interference spectrum signal based on the k scales comprises: decomposing the any interference spectrum signal based on each of the k scales, to obtain k scale-transformed signals corresponding to the any interference spectrum signal;

a frequency domain transforming module configured to perform Fourier transform on each of the scale-transformed signals and take a logarithm, to obtain an axial frequency domain signal in a logarithmic space, and denoise the axial frequency domain signal;

a decorrelation module configured to divide, based on the k scales, N $\times$ k of the axial frequency domain signals denoised into k sets of single-scale signals, and perform decorrelation calculation on each set of the single-scale signals to obtain single-scale blood flow signals;

a fusion module configured to obtain a multi-scale blood flow signal based on k sets of the single-scale blood flow signals; and

an imaging module configured to obtain a blood flow image based on the multi-scale blood flow signal.

According to the above technical solution, the blood flow signal is a non-stationary signal, and its changes are localized. An interference spectrum signal is decomposed into multiple segments after a scale transform. In the subsequent decorrelation calculation, axial frequency domain signals corresponding to each segment of the interference spectrum signal corresponding to the same target area at different times can be compared. Through segmented comparison, changing signals in a non-stationary state can be captured more accurately, to thus implement accurate extraction of blood flow signals.

Moreover, multi-scale transform is performed on an interference spectrum signal, and the amount of decomposed segments corresponding to each scale is different, i.e., the scales of segmented comparison are different, such that the changes of non-stationary signals can be measured at different scales. Therefore, simultaneously performing scale transform and fusion of multiple scales on an interference spectrum signal can reduce the misjudgment rate of a single scale while improving the accuracy and processing efficiency of blood flow signal extraction; and as all the points in the interference spectrum signal are reserved, more details of the image can be restored, thus improving the signal-to-noise ratio.

In a possible implementation, the scale-transforming module decomposes the any interference spectrum signal based on any scale, to obtain scale-transformed signals, which is specifically configured to:

the interference spectrum signal corresponding to a sampling time period T;

determine decomposed signals corresponding to the any scale, the decomposed signals being signals within the sampling time period T, the decomposed signals comprising at least one time set that includes a first time period and a second time period adjacent to each other, two of the first time periods adjacent to each other, and two of the second time periods adjacent to each other;

multiply the interference spectrum signal corresponding to the first time period by a forward signal to obtain a first signal;

multiply the interference spectrum signal corresponding to the second time period by a reverse signal to obtain a second signal; and obtain scale-transformed signals based on the at least one first signal and the at least one second signal.

In a possible implementation, the apparatus further comprises a denoising module configured to denoise the axial frequency domain signals within the logarithmic space, to obtain denoised axial frequency domain signals, which is specifically configured to:

determine a segmentation threshold;

if an amplitude of an axial frequency domain signal is greater than or equal to the segmentation threshold, the denoised axial frequency domain signal is equal to the axial frequency domain signal in the logarithmic space; and

if the amplitude of the axial frequency domain signal is less than the segmentation threshold, the denosed axial frequency domain signal is equal to the segmentation threshold.

In a possible implementation, the denoising module determines the segmentation threshold, which is specifically configured to:

extract noise signals corresponding to all the axial frequency domain signals based on a clustering algorithm; and

determine the segmentation threshold based on the noise signals.

In a possible implementation, the decorrelation module performs decorrelation calculation on each set of the single-scale signals to obtain the single-scale blood flow signals, which is specifically configured to:

determine first-order statistics, second-order statistics, and third-order statistics of each set of the single-scale signals; and

perform the decorrelation calculation based on the first-order statistics, the second-order statistics, and the third-order statistics to determine the single-scale blood flow signals.

In a possible implementation, the apparatus further comprises an alignment module which is specifically configured to: after obtaining the axial frequency domain signals in the logarithmic space,

perform an alignment operation on all the axial frequency domain signals based on a phase correlation algorithm;

wherein the denoised axial frequency domain signals are signals obtained by denoising the axial frequency domain signals after the alignment operation.

In a possible implementation, the apparatus further comprises a dispersion compensation module which is specifically configured to:

prior to performing scale transform on the respective interference spectrum signals in the N interference spectrum signals based on the k scales, perform dispersion compensation on the respective interference spectrum signals in the time domain signals.

In a third aspect, the present application provides an electronic device, specifically as follows:

An electronic device, comprising:

one or more processors; and

a memory;

wherein one or more computer programs are stored in the memory and configured to be executed by the one or more processors, and the one or more computer programs are configured to execute the method for optical coherence tomography angiography as described above.

In a fourth aspect, the present disclosure provides a computer readable storage medium, specifically as follows:

A computer readable storage medium, comprising a computer program that can be loaded by a processor and configured to execute the method for optical coherence tomography angiography as described above.

In view of the above, the present disclosure has at least the following advantageous technical effects:

An interference spectrum signal is decomposed into multiple segments after a scale transform. In the subsequent decorrelation calculation, axial frequency domain signals corresponding to each segment of the interference spectrum signal corresponding to the same target area at different times can be compared. Through segmented comparison, changing signals in a non-stationary state can be captured more accurately, to thus implement accurate extraction of blood flow signals. As all the points in the interference spectrum signal are reserved, more details of the image can be restored, thus improving the signal-to-noise ratio. Further, multi-scale transform is performed on an interference spectrum signal, and the amount of decomposed segments corresponding to each scale is different, i.e., the scales of segmented comparison are different, such that the changes of non-stationary signals can be measured at different scales. Therefore, simultaneously performing scale transform and fusion of multiple scales on an interference spectrum signal can reduce the misjudgment rate of a single scale while improving the accuracy and processing efficiency of blood flow signal extraction.

## BRIEF DESCRIPTION OF THE DRAWINGS

Fig. 1 is a schematic flowchart of a method of optical coherence tomography angiography according to embodiments of the present disclosure;

Fig. 2 is a schematic diagram illustrating multi-scale transform of a time domain signal;

Fig. 3 illustrates: (a) a blood flow image of glass membrane; (b) a deep layer image; and (c) a superficial blood flow image provided by embodiments of the present disclosure;

Fig. 4 is a schematic flowchart of steps S021 through S023 according to embodiments of the present disclosure;

Fig. 5 is a schematic flowchart of steps S031 through S033 according to embodiments of the present disclosure;

Fig. 6 is a schematic diagram illustrating performing decorrelation calculation based on single-scale signals;

Fig. 7 is a schematic diagram illustrating an apparatus for optical coherence tomography angiography according to embodiments of the present disclosure; and

Fig. 8 is a schematic diagram illustrating an electronic device according to embodiments of the present disclosure.

## DETAILED DESCRIPTION OF EMBODIMENTS

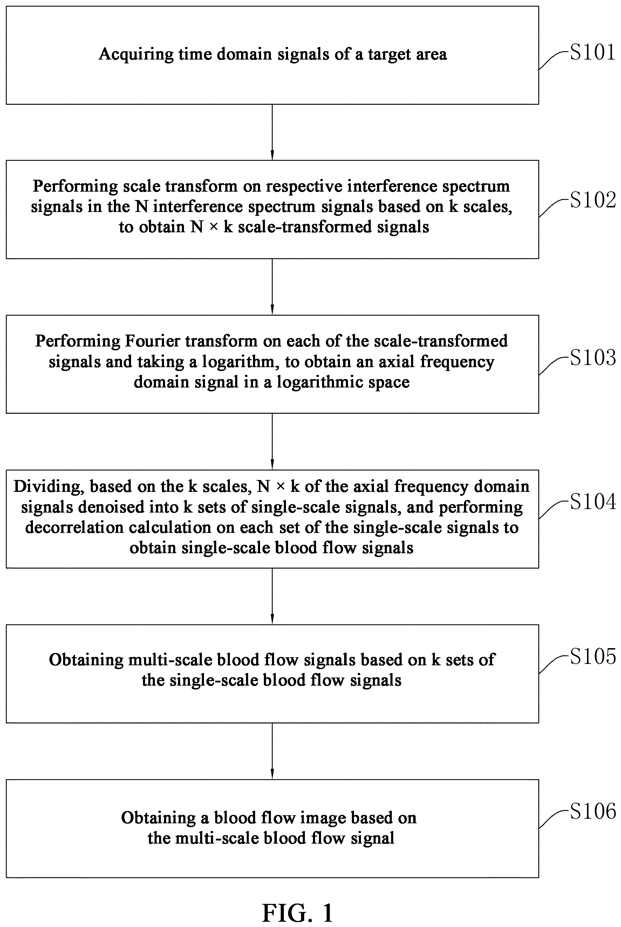

Reference will now be made to Figs. 1-8 to further describe the present disclosure in detail.

After reading this Description, those skilled in the art may make modifications to the embodiments as required, without making creative contribution, and as long as falling within the scope of the claims of the present disclosure, they are all protected according to the patent law.

In order to make the objective, technical solution and advantages of the embodiments of the present disclosure more apparent, clear and full description on the technical solution of the embodiments of the present disclosure will be provided below with reference to the accompanying drawings. It is to be understood that only a part of embodiments of the present disclosure will be described here, rather than all of them. All the other embodiments obtained by the ordinary skilled in the art on the basis of the embodiments described here without creative efforts, should fall into the protection scope of the present disclosure.

In addition, the term "and/or" used here only describes an association relationship of related objects, indicating that there may be three relationships. For example, A and/or B may indicate the following three relationships: presence of A only, simultaneous presence of A and B, and presence of B only. Moreover, the sign "/" used here generally refers to that the related objects preceding and after the sign are in an "or" relationship, unless indicated otherwise.

For convenience in understanding the technical solution of the present disclosure, a plurality of elements will be introduced in the description of the present disclosure. It would be appreciated that the following introduction is only provided for easy understanding of those elements, to further make the present disclosure more apparent, without covering all possibilities.

(1) A-scan: refers to a depth reflection profile reflected from a sample in a depth direction, wherein one interference spectrum signal is obtained through one A-scan, where image information of the sample in an axial depth is all included in the interference spectrum signal, and an axial depth image of a sample can be obtained through its Fourier Transform.

(2) B-scan: includes a plurality of continuous, adjacent interference spectrum signals (A-scan signals), for example, N1 interference spectrum signals each including X points, where these spectra form an $N1 \times X$ data packet. Specifically, for the time-domain optical coherence tomography, scanning in the depth direction is required, which is not conductive to high-speed real-time imaging of OCT. In contrast, a frequency-domain optical coherence tomography system can obtain all information (A-scan signals) in a depth direction (a Z direction) by performing Fourier transform on coherent spectra of a sample arm and a reference arm, wherein only transverse scans (scans in an X and a Y direction) are required, without mechanical scans (A-scans) in the depth direction, where a B-scan image is formed through a scan in the X direction and an OCT three-dimensional signal is formed through a scan in the Y direction.

(3) Axial resolution of OCTA system: refers to a minimum distance that can be resolved for axial depth tomography of a sample, where X points included in each inherence spectrum signal are determined by the axial resolution of the OCTA system, wherein the transverse resolution and the axial resolution of the OCTA image are not related to each other and the OCTA system can provide the axial resolution (approximately 1-15 $\mu$m) independent of the transverse resolution, which axial resolution is mainly dependent on a coherence length of a width light source.

(4) Signal-to-noise ratio: refers to an index for image evaluation by calculating ration of signal to noise in an image, which is a global evaluation criterion. For an OCTA image, the greater the signal-to-noise ratio, the less speckle noise in the image, which means a better image denoising effect and more satisfactory image quality.

In embodiments of the present disclosure is a method of optical coherence tomography angiography provided, which is executed by an electronic device. With reference to Fig. 1, the method includes:
Step S101, acquiring time domain signals of a target area.

In which, the time domain signals are N interference spectrum signals obtained by performing N A-scans on the target area, and are expressed as I (i, x, t), where i = 1, 2, ... N, x is a scanned position (i.e., the target area), t is a scanning time point of a current A-scan, wherein a duration corresponding to each A-scan is a sampling time period T and a scan rate of an A-scan is generally set to 7,000 times per second, 10,000 times per second or the like.

The depth information of a sample can be obtained by performing Fourier transform on interference spectrum signals collected through one A-scan. If an interference spectrum signal corresponds to more data points, the amount of points in the Fourier transformed image is greater, resulting in a higher resolution of the image. For angiography, an image with a higher resolution enables more accurate identification of blood flow signals in different tissues and layers.

The duration of a single A-scan (the sampling time period T) is set artificially. For setting the duration of the single A-scan, the following information should be taken into account: an incident light power, a sample reflectivity, a measurement depth, a depth information acquisition speed, and the like. For example, in a weakly scattering medium, the sensitivity of the OCT system determines a minimum detectable light signal; in a strongly scattering medium, the sensitivity of the OCT system determines a maximum imaging depth, wherein the sensitivity of the OCT system can in turn be improved by prolonging the signal collection time of the single A-scan and reducing the depth information acquisition speed.

For analysis of stationary signals, Fourier transform is the primary analysis method which is also the one mostly commonly used. However, as the Fourier transform is a global transform (corresponding to a sampling time period T), a local time-frequency property, namely the most fundamental property of a non-stationary signal, cannot be expressed. The blood flow signal interested in the embodiments of the present disclosure is exactly the type of non-stationary signal.

In order to extract changes of a blood flow signal, an improved algorithm is proposed in the split-spectrum amplitude-decorrelation angiography (SSADA-OCT), where the SSADA divides the entire OCT spectrum into a plurality of narrow bands, and correlations between different B-scans are calculated respectively for the plurality of bands and then averaged. The specific steps include: during data processing, separating the spectra using bandpass filters at different positions (e.g. 4 bandpass filters spaced equidistantly), which is called as a step of spectrum separation; thereafter obtaining M independent decorrelated images based on each pair of B-scans, where the images obtained have a low axial resolution equal to the transverse resolution.

However, although more accurate changes of the blood flow signal can be obtained with the spectrum separation approach as described above, the signal-to-noise ratio will be increased and some data points in the interference spectrum signal are discarded, thereby causing the reduced axial resolution of the restored image.

In order to retain the high axial resolution while more accurately extracting a changed blood flow signal, according to the embodiments of the present disclosure, all data points corresponding to an A-scan are reserved, wherein the signal corresponding to the A-scan is decomposed into multiple segments of unit signals (i.e., a scale transform), and changes of the blood flow signal within each segment of the unit signal is analyzed to thus extract the blood flow signal accurately.

Step S102, performing scale transform on respective interference spectrum signals in the N interference spectrum signals based on k scales, to thus obtain N × k scale-transformed signals.

In which, performing scale transform on any interference spectrum signal based on k scales includes: decomposing the any interference spectrum signal based on each scale, to thus obtain k scale-transformed signals corresponding to the any interference spectrum signal.

For a scale transform on the interference spectrum signal, the interference spectrum signal is decomposed into multiple segments of unit signals. During decorrelation calculation, the axial frequency domain signal corresponding to each segment of unit signal can be compared. Through segmented comparison, the non-stationary changing signal in an interference spectrum signal can be captured more accurately, to achieve accurate extraction of the blood flow signal.

Multi-scale transform is performed on an interference spectrum signal, and the amount of decomposition segments corresponding to each scale is different, i.e., the scales of the segmental comparison are different, such that the changes of non-stationary signal can be measured at different scales.

Compared with the spectrum separation approach where repeated calculation leads to low calculation efficiency since the frequency bands may overlap during spectrum separation, the embodiments of the present disclosure can reserve all points in an interference spectrum signal while avoiding repeated calculation of data.

Step S103, performing Fourier transform on each scale-transformed signal and taking a logarithm, to obtain an axial frequency domain signal in the logarithmic space.

Step S104, dividing the N × k denoised axial frequency domain signals into k sets of single-scale signals, and performing decorrelation calculation on each set of single-scale signals, to thus obtain single-scale blood flow signals.

According to embodiments of the present disclosure, the principle of denoising the axial frequency domain signal is described below: the quantitative effect of the decorrelation algorithm on the movement contrast has a significant dependence on the noise level of the original A-scan signal; with attenuation of the signal intensity (e.g., in a deep tissue area), random noise will gradually become the main component and a great decorrelation value will be generated, resulting in decorrelation artifacts. For example, the randomness of noise and the decorrelation caused by the movement of red blood cells cannot be distinguishing from each other based on the movement contrast generated through the decorrelation calculation, so that the area with a weak signal-to-noise ratio is easily misjudged as a blood flow signal area, which seriously affects the contrast of the blood flow image. After denoising, the denoised axial frequency domain signals can reduce or eliminate the error caused by the random noise signals in the subsequent decorrelation calculation.

Step S105, obtaining multi-scale blood flow signals based on the k sets of single-scale blood flow signals.

In which, after a B-scan signal including a plurality of A-scan signals at different positions is processed in steps S101 through S104, the A-scan signal at any position is a multi-scale blood flow signal corresponding to the any position; by analyzing a scale blood signal at each position in a B-scan signal, a blood vessel signal of the entire structure section can be obtained.

According to embodiments of the present disclosure, by simultaneously performing scale transform and fusion of multiple scales on an interference spectrum signal initially acquired, both the blood flow signal extraction accuracy and processing efficiency can be improved.

Step S106, obtaining a blood flow image based on the multi-scale blood flow signal.

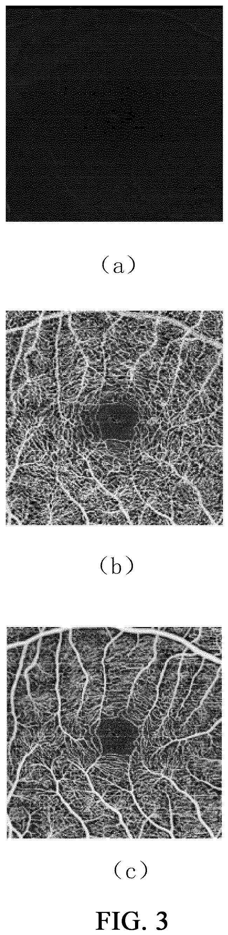

Specifically, visualization software is used to transform the multi-scale blood flow signal in to a blood vessel image; Fig. 3 shows (a) glass membrane blood flow image; (b) a deep layer image; and (c) superficial blood flow image, respectively corresponding to the fused multi-scale blood flow signal. Through fusion of multiple scales, the present disclosure can reduce the misjudgment rate at a single scale, improve the signal-to-noise ratio, and enable blood flow signals corresponding to different tissues and layers to be accurately identified.

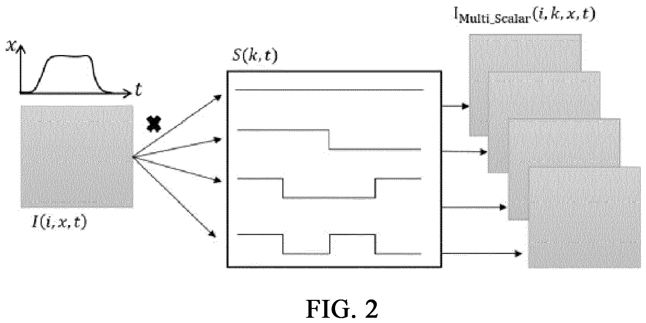

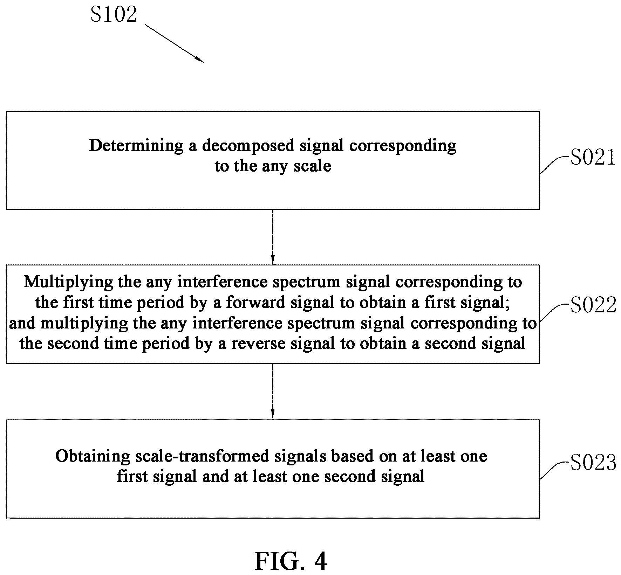

According to a possible implementation of the embodiments of the present disclosure, referring to Figs. 2 and 4, in step S102, any interference spectrum signal can be decomposed based on any scale, to obtain the scale-transformed signal, which includes:

Step S021, determining decomposed signals corresponding to the any scale.

In which, each scale corresponds to a preset decomposed signal which is expressed in the following form:

$$[1,1,-1,-1]$$

$$[1,1,-1,-1]$$

$$[1,-1,-1,1]$$

$$[1,-1,1,-1]$$

$$\ldots$$

In which, the decomposed signal is the signal within the sampling time period T, which includes at least one time set including a first time period and a second time period adjacent with each other, two adjacent first time periods, or two adjacent second time periods.

[1,1,-1,-1] is taken as an example for illustration. The decomposed signal corresponds to two time sets, specifically a first time set (T1, T1) and a second time set (T2, T2), where the first time set is located preceding the second time set within the sampling time period T.

[1,-1,-1,1] is taken as a further example. The decomposed signal corresponds to two time sets, specifically a first time set (T1, T2) and a second time set (T2, T1), where the first time set is located preceding the second time set within the sampling time period T.

Further, for ease of representing k decomposed signals, the k decomposed signals corresponding to k scales may be arranged in rows to form a non-zero orthogonal signal S which may be expressed as follows:

$$S(j,t) = \text{Walsh}(k) \otimes [1,1,\ldots,1,1]_{1\times(M/k)} \qquad \text{Formula (1)}$$

Wherein, Walsh(k) is a k-dimensional Walsh transformation matrix; $\otimes$ is a Kronecker product; M is an amount of current A-scan sampling points; j = 1, 2, ... k; t is a time scale of the signal.

For example, when k = 4,

$$\text{Walsh}(4) = \begin{bmatrix} 1 & 1 & 1 & 1 \\ 1 & 1 & -1 & -1 \\ 1 & -1 & -1 & 1 \\ 1 & -1 & 1 & -1 \end{bmatrix}.$$

Step S022, multiplying the interference spectrum signal corresponding to the first time period by a forward signal to obtain a first signal, and multiplying the interference spectrum signal corresponding to the second time period by a reverse signal to obtain the second signal.

The first signal and the second signal are different. Referring to Fig. 2, the decomposed signal illustrated in the embodiments of the present disclosure is a rectangular wave signal. In the embodiments of the present disclosure, the first signal and the second signal also include signals in other forms. For example, smooth transition may be performed between the first signal and the second signal.

Continuing with the above example, 1 is a forward signal, while -1 is a reverse signal, and T1 corresponds to the forward signal, while T2 corresponds to the reverse signal.

Step S023, obtaining scale-transformed signals based on at least one first signal and at least one second signal.

The scale-transformed signals obtained are expressed below by Formula (2):

$$I(i, x, t) * [1, 1, -1, -1] \qquad \text{Formula (2)}$$

Further, the N × k scale-transformed signals obtained by performing scale transform on respective interference spectrum signals in the N interference spectrum signals based on k scales may be expressed as follows:

$$I_{Multi\_Scalar}(i, j, x, t) = I(i, x, t) * S(j, t) \qquad \text{Formula (3)}$$

Wherein, $I_{Multi\_Scalar}(i,j,x,t)$ includes N × k scale-transformed signals.

According to the embodiments of the present disclosure, different scales and decomposed signals corresponding to the respective scales may be set for different application requirements. After scale transform is performed, each interference spectrum signal corresponds to k scale-transformed signals, and N × k scale-transformed signals are obtained for N interference spectrum signals.

As a possible implementation of embodiments of the present disclosure, step S101 further includes: performing dispersion compensation for the respective interference spectrum signals in the time domain signal, after acquiring the time domain signals of the target area and before performing scale transform on the respective interference spectrum signals in the N interference spectrum signals based on k scales.

The axial resolution of the OCT system is inversely proportional to the half-maximum width of the interference spectrum signal. In an actual system optical path, the difference between lengths of the dispersion materials of the reference arm and the sample arm may cause dispersion mismatch, and phase changes are introduced into the interference spectrum signal to broaden the spectrum of the interference spectrum signal, which may deteriorate the axial resolution of the OCTA image and reduce the signal-to-noise ratio.

The known dispersion mismatch comes from system components including: a collimator, a scanning objective lens, an optical fiber and a sample to be tested. The sample at different depths is different with respect to length of the dispersion medium through which the probe light passes, and amount of dispersion mismatch, so that the sample at different depths has a different axial resolution accordingly. In addition, k-nonlinear acquisition of spectral signals will also introduce a phase factor related to the optical path difference between the two arms and a wave vector k, which has the same impact as the dispersion mismatch. As such, in order to keep the axial resolution close to the theoretical axial resolution at different depths within the imaging depth range, it is especially important to compensate for the dispersion as the depth changes.

At present, the dispersion compensation methods of the OCTA system can be divided into two types: physical compensation and numerical compensation. The physical compensation method includes compensating dispersion mismatch between two arms by adding hardware devices in the reference arm. The physical compensation method requires addition and modification of hardware devices in the optical path, which may complicate the system, introduce additional noise, and increase costs. By comparison, the numerical compensation method only needs to perform subsequent processing on the data collected by OCTA to eliminate dispersion mismatch, which is employed in the embodiments of the present disclosure to implement dispersion compensation on the time domain signal.

Specifically, the numerical compensation methods are mainly of the following types:

The first is the iterative compensation method, which includes iteratively finding a dispersion compensation coefficient having the compensation effect using spatial image quality of the A-scan (e.g. a sharpness, a Rayleigh entropy, and the like) as the evaluation function. Marks *et al.* used a golden section-based iterative method to compensate for dispersion to improve the resolution of the OCTA system.

The second is the deconvolution method, which includes calculating the dispersion parameter convolution kernel that changes with the depth of the sample based on the structure and the material of the sample, and eliminating the dispersion mismatch through deconvolution operations.

The third is the short-time Fourier transform method, which includes dividing the spectrum using a window function, and calculating the dispersion phase that should be compensated for light at different frequencies.

The fourth is the spatial domain extraction and fitting method, which includes setting a window function in the spectrum of the interference spectrums signal according to a certain threshold, extracting Gaussian peak signals at different steps of the sample, and obtaining, with the fitting approach, and then removing second-order and third-order dispersion terms representing dispersion mismatch in the corresponding spectrum.

The fifth is the dispersion coefficient fitting method, which includes calculating the dispersion coefficients of the sample at different depths in advance, using an iterative method, and thus obtaining material information of the sample and further compensating for a weak signal at a deeper position of the sample.

In the embodiments of the present disclosure, after obtaining the updated time domain signals by performing dispersion compensation in step S101, scale transform is performed in step S 102 on the respective interference spectrum signals in the dispersion-compensated time domain signal, based on k scales, to thus obtain N × k scale-transformed signals.

During OCTA data collection, it is necessary to continuously collect, at the same position, B-scans having a certain time interval therebetween, and reconstruct microvascular images by comparing the decorrelation signals between B-scans. The data collection process is not free from the influence of sample movement, environmental noise, system vibration, and the like. In the case, the data may be discontinuous, which is shown as distortions and breaks in blood vessel images, causing movement artifacts generated in the images. The artifacts caused by sample movements may heavily deteriorate the imaging quality of OCTA and affect the quantitative research on tissue microvessels.

There are many sources of sample movements, such as breathing and heartbeat, involuntary shaking of the measured part, and the like. Even small movements, for example, micron-level movements in the skin caused by heartbeat, may increase background noise and eventually affect blood vessels. The longer the time interval between data collections, the more serious the movement artifact. Therefore, in the OCTA blood vessel extraction algorithm, elimination of movement artifacts is key, and the main method for artifact elimination is image registration.

Specifically, image registration includes transformation, interpolation, displacement, and the like, with the purpose of correcting distortions, breaks, and the like caused by movements in an image, and obtaining an image without movement artifacts.

According to embodiments of the present disclosure, the main factor affecting reduction of the signal-to-noise ratio is translation. Therefore, as a possible implementation of the embodiments of the present disclose, step S 103 further includes: after performing Fourier transform on each scale-transformed signal and taking a logarithm, to obtain an axial frequency domain signal in the logarithmic space, aligning all axial frequency domain signals based on a phase correlation algorithm; wherein the denoised axial frequency domain signals are signals obtained by denoising the frequency domain signals after the alignment operation.

Image translation may be handled using a phase correlation algorithm, and the Fourier-Mellin (FMT) algorithm combined with a logarithmic polar coordinate model is an extension of the Fourier phase correlation algorithm. With the FMT, rotated and scaled images can be directly processed. In the frequency domain-based method, registration is performed using all content information of the image.

Specifically, the process of the phase correlation algorithm for translation processing is:

Assumed that $f_1(x,y)$ and $f_2(x,y)$ only have a translation $(x_0,y_0)$:

$$f_2(x,y)=(x-x_0,y-y_0) \qquad \text{Formula (4)}.$$

After the Fourier transform,

$$F_2(\xi,\eta) = e^{-j2\pi(\xi_0+1\eta_0)} * F_1(\xi,\eta) \qquad \text{Formula (5)}$$

A cross power spectrum of two images is:

$$e^{-j2\pi(\xi x_0 + \nu_0)} = \frac{F_1(\xi,\eta)F_2^*(\xi,\eta)}{|F_1(\xi,\eta)F_2^*(\xi,\eta)|} \qquad \text{Formula (6)}.$$

Wherein, $F_2^*(\xi,\eta)$ is a complex conjugate of $F_2(\xi,\eta)$. Formula (6) is subjected to two-dimensional inverse Fourier transform to obtain an impulse function, which is approximately 1 at $(x_0, y_0)$, and approximately 0 at other positions. In this way, the translation parameters $(x_0, y_0)$ can be determined.

All the axial frequency domain signals in the logarithmic space obtained in step S103 are aligned based on the above phase correlation algorithm, to obtain all the aligned axial frequency domain signals. Then all the aligned axial frequency domain signals are denoised to obtain the denoised axial frequency domain signals.

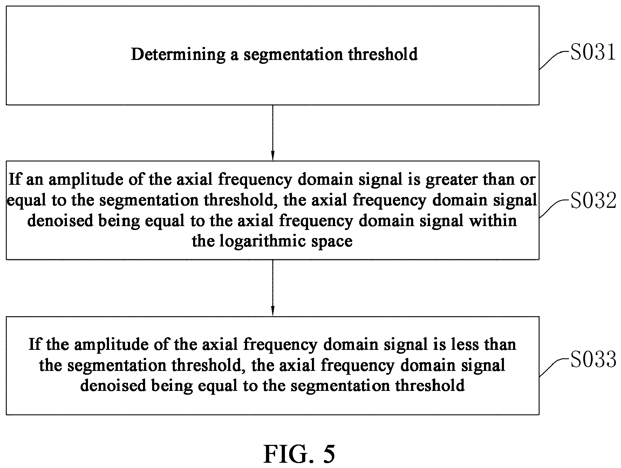

As a possible implementation of the embodiments of the present disclosure, referring to Fig. 5, after step S103, denosing the axial frequency domain signals in the logarithmic space to obtain the denoised axial frequency domain signals includes;

Step S031, determining a segmentation threshold.

The noise reduction method for decorrelation artifacts includes: when counting A-scan signal changes, setting an intensity threshold to isolate the impact of noise signals on OCTA, and removing all signals with a low signal-to-noise ratio by generating an intensity mask, which can reduce or eliminate the error caused by the random noise signal in subsequent decorrelation calculation. The segmentation threshold in the embodiments of the present disclosure is the intensity mask. Typically, the segmentation threshold is set empirically. In order to further improve the accuracy, according to embodiments of the present disclosure, determining the segmentation threshold includes: extracting noise signals corresponding to all axial frequency domain signals based on a clustering algorithm; and determining the segmentation threshold based on the noise signals.

The clustering method accomplishes the purpose of removing the noise signals by finding outliners from clusters in the signals and then removing them. In the embodiments of the present disclosure, signals falling out of the cluster are noise signals.

The K-means algorithm is a typical distance-based clustering algorithm, where K is taken as a parameter input, K center points are selected randomly, and n objects are eventually grouped into K clusters. Members from the same cluster in the K clusters have a high similarity, and members from different clusters have a high dissimilarity. Specifically, a cluster center in the K-means clustering algorithm is determined by calculating a mean value of all signal object attributes in a cluster, and a distribution of noised signal classes can be obtained by performing K-means cluster analysis on the image signal intensity.

Specifically, the process of extracting noise signals based on the K-means clustering algorithm includes step S11 (not shown), step S12 (not shown), step S13 (not shown), step S14 (not shown) and step S15 (not shown), specifically:

Step S11, selecting K cluster centers as current cluster centers using a farthest preference strategy, where K is a natural number;

Step S12, clustering all the axial frequency domain signals according to the current clustering centers, and clustering each axial frequency domain signal into a cluster represented by a clustering center closest thereto;

Step S12, calculating a mean value of each current cluster as a new cluster center;

Step S13, determining whether the new cluster center is the same as the previous clustering center, and if yes, performing step S14, or if no, using the new cluster center as the current cluster center and repeating steps S12 and S13;

Step S14, calculating a distance between any two cluster centers in all the new cluster centers; and

Step S15, determining whether the distance between any two cluster centers is greater than a set reference threshold, and if yes, filtering out clusters each having a distance between any two cluster centers greater than the set reference threshold, and using the axial frequency domain signals corresponding to the filtered clusters as noise signals, or if no, outputting information indicating absence of noise signals.

After the noise signals are obtained, a mean and a variance of the noise signals can be obtained by performing distribution statistics on the noise signals, and the segmentation threshold can then be determined based on the mean and the variance. Specifically, the method for selecting an optical threshold includes: a maximum inter-class variance method. The method for determining a threshold based on a mean and a variance is known to those skilled in the art, details of which are omitted there for brevity.

After the segmentation threshold is determined, the axial frequency domain signal can be segmented based on the signal intensity corresponding to the segmentation threshold, to separate an effective target from noise to thus improve the signal-to-noise ratio of the image.

Step S032, if the amplitude of the axial frequency domain signal is greater than or equal to the segmentation threshold, the denoised axial frequency domain signal is equal to an axial frequency signal in the logarithmic space.

Step S033, if the amplitude of the axial frequency domain signal is less than the segmentation threshold, the denoised axial frequency domain signal is equal to the segmentation threshold.

Steps S032 and S033 are specifically expressed as follows:

$$\hat{F}_{Multi\_Scalar}(i,j,x,z) = \begin{cases} F_{Multi\_Scalar}(i,j,x,z), & F_{Multi\_Scalar}(i,j,x,z) > th \\ th, & F_{Multi\_Scalar}(i,j,x,z) \le th \end{cases} \qquad \text{Formula (7)}$$

Wherein, $\hat{F}_{Multi\_Scalar}(i, j, x, z)$ is a denoised axial frequency domain signal in the logarithmic space; *th* is a segmentation threshold; j is any scale in k scales; and $F_{Multi\_Scalar}(i, j, x, z)$ is an axial frequency domain signal in the logarithmic space.

Subsequent to obtaining the denoised axial frequency domain signal, differential analysis on adjacent B-scan images is performed based on the principle of the decorrelation algorithm, using the signal correlation change caused by the blood flow, to extract blood flow signals.

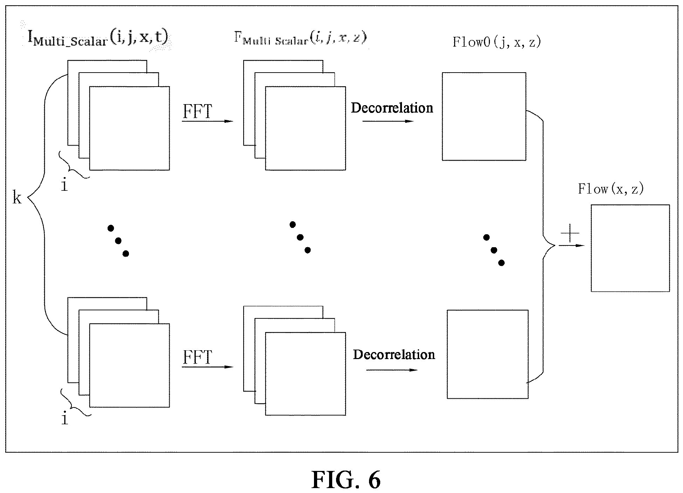

As a possible implementation of the embodiments of the present disclosure, referring to Fig. 6, performing decorrelation calculation on each set of single-scale signals to obtain single-scale blood flow signals in step S105 includes: determining first-order statistics, second-order statistics, and third-order statistics for each set of single-scale signals; and performing decorrelation calculation based on the first-order statistics, the second-order statistics, and the third-order statistics, to determine single-scale blood flow signals.

Wherein,

The first-order statistics is:

$$J_1(j,x,z) = 1/N \sum_i \hat{F}_{\text{Multi\_Scalar}}(i,j,x,z) \quad \text{Formula (8)};$$

The second-order statistics is:

$$J_2(j,x,z) = 1/N \sum_i \hat{F}_{\text{Multi\_Scalar}}(i,j,x,z)^2 \quad \text{Formula (9)};$$

and
The third-order statistics is:

$$J_3(j,x,z) = 1/N \sum_i \hat{F}_{\text{Multi\_Scalar}}(i,j,x,z)^3 \quad \text{Formula (10)}.$$

From the statistical perspective, for a random variable (vector) from a normal distribution, its statistical characteristics can be fully expressed by first-order and second-order statistics. For example, for a random vector from a Gaussian distribution, if its mathematical expectation and covariance matrix are known, its joint probability density function can be obtained. For a Gaussian random process, if the mean and autocorrelation function (or autocovariance function) are known, its probability profile, namely entire statistical characteristics, can be obtained.

However, when the random variable or random process does not follow the Gaussian distribution, the first-order and second-order statistics cannot fully represent its statistical characteristics; or information is not completely included in the first-order and second-order statistics, and a large amount of useful information is included in higher-order statistics.

The main motivations for using high-order statistics during signal processing include: 1) suppressing the effect of additive colored noise of an unknown power spectrum; 2) identifying non-minimum phase systems or reconstructing non-minimum phase signals; 3) extracting various information caused by Gaussian deviation; and 4) detecting and characterizing nonlinearities in signals and nonlinear systems.

Therefore, the higher-order statistics signal processing method is a method o extracting useful information of the signals from the high-order statistics of non-Gaussian signals, in particular when information cannot be extracted from the first-order and second-order statistics. From this perspective, the high-order statistics method is not only an important supplement to the correlation function-based or power spectrum-based random signal processing method, but also can provide means against lots of signal processing problems that cannot be solved by two-order statistics method.

Performing decorrelation calculation based on the first-order statistics, the second-order statistics, and the third-order statistics, to determine the single-scale blood flow signals, includes:

$$\begin{aligned}
\text{Flow0}(j,x,z) &= \alpha_1 J_3(j,x,z) + \alpha_2 J_2(j,x,z) * J_1(j,x,z) + \alpha_3 J_1(j,x,z)^3 \\
&+ \alpha_4 J_2(j,x,z) + \alpha_5 J_1(j,x,z)^2
\end{aligned} \quad \text{Formula (11)}$$

In consideration of a sum of the cubic variance and the 2-fold variance of the blood flow signal, it is employed by the following formula:

$$1/N \sum_i [\hat{F}_{Multi\_Scalar}(i,j,x,z) - mean \ (\hat{F}_{Multi\_Scala}$$

$$= J_3(j,x,z) - 3J_2(j,x,z)J_1(j,x,z) + 2J$$

$$2/N \sum_i [\hat{F}_{Multi\_Scalar}(i,j,x,z)$$

$$- mean \ (\hat{F}_{Multi\_Scalar}(i,j,x,.$$

$$= 2 * J_2(j,x,z) - 2 * J_1(j,x,z)^2 \qquad \text{Formula (12)}$$

Thus, $[\alpha 1, \alpha_2, \alpha_3, \alpha_4, \alpha_5]$ can be valued as [1, -3, 2, 2, -2].

As a possible implementation of the embodiments of the present disclosure, obtaining multi-scale blood flow signals based on the k sets of single-scale blood flow signals in step S106 includes: superimposing the k sets of single-scale blood flow signals to obtain multi-scale blood flow signals.

$$Flow(x,z) - \sum_j{}' Flow0(j,x,z) \qquad \text{Formula (13)}$$

According to the above-mentioned embodiments, the method for optical coherence tomography angiography has been introduced from the perspective of the process flow, and an apparatus for optical coherence tomography angiography 100 will be described below in detail from the perspective of virtual modules or virtual units according to the following embodiments.

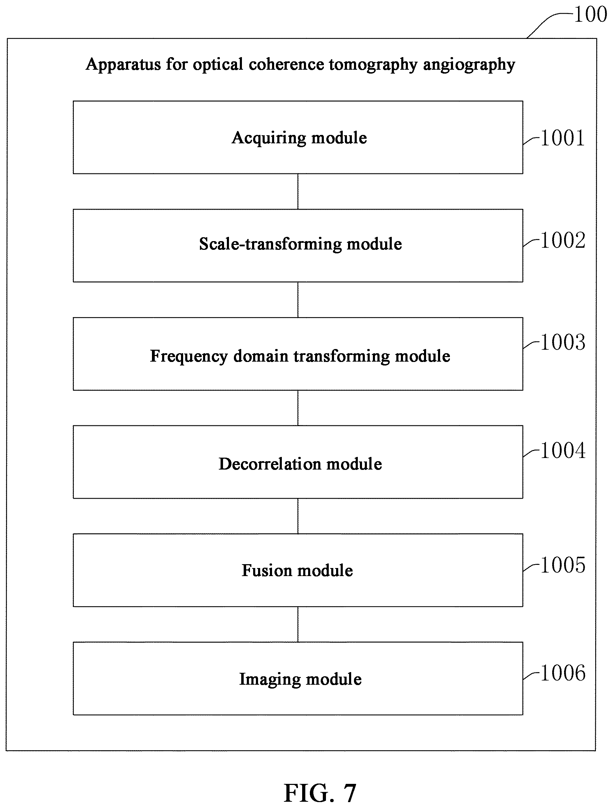

The embodiments of the present disclosure provide an apparatus for optical coherence tomography angiography 100. Referring to Fig. 7, the apparatus for optical coherence tomography angiography 100 includes:

an acquiring module 1001 configured to acquire time domain signals of a target area, where the time domain signals are N interference spectrum signals obtained by repeating A-scans on the target area N times;

a scale-transforming module 1002 configured to perform scale transform on respective interference spectrum signals in the N interference spectrum signals based on k scales, to obtain N $\times$ k scale-transformed signals;

wherein performing scale transform on any interference spectrum signal based on the k scales includes decomposing the any interference spectrum signal based on each scale, to obtain k scale-transformed signals corresponding to the any interference spectrum signal;

a frequency domain transforming module 1003 configured to perform Fourier transform on each scale-transformed signal and take a logarithm, to obtain an axial frequency domain signal in a logarithmic space;

a decorrelation module 1004 configured to divide N $\times$ k denoised axial frequency domain signals into k sets of single-scale signals based on k scales, and performing decorrelation calculation on each set of single-scale signals to obtain single-scale blood flow signals;

a fusion module 1005 configured to obtain multi-scale blood scale signal based on the k sets of single-scale blood flow signals; and

an imaging module 1006 configured to obtain a blood flow image based on the multi-scale blood flow signals.

According to the above technical solution, the blood flow signal is a non-stationary signal, and its changes are localized. An interference spectrum signal is decomposed into multiple segments after a scale transform. In the subsequent decorrelation calculation, axial frequency domain signals corresponding to each segment of the interference spectrum signal corresponding to the same target area at different times can be compared. Through segmented comparison, the changing signals in a non-stationary state can be captured more accurately, to thus implement accurate extraction of blood flow signals.

Moreover, multi-scale transform is performed on an interference spectrum signal, and the amounts of decomposed segments corresponding to each scale are different, i.e., the scales of segmented comparison are different, such that the changes of non-stationary signals can be measured at different scales. Therefore, simultaneously performing scale transform and fusion of multiple scales on an interference spectrum signal can reduce the misjudgment rate of a single scale while improving the accuracy and processing efficiency of blood flow signal extraction; and as all the points in the interference spectrum signal are reserved, more details of the image can be restored, thus improving the signal-to-noise ratio.

In a possible implementation, the scale-transforming module 1002 decomposes any interference spectrum signal based on any scale when the interference spectrum signal is corresponded to a sampling time period T, to obtain scale-transformed signals, which is specifically configured to:

determine decomposed signals corresponding to the any scale, where the decomposed signals are signals within the sampling time period T, and the decomposed signals include at least one time set that includes a first time period and a second time period adjacent to each other, two first time periods adjacent to each other, and two second time periods adjacent to each other;

multiply an interference spectrum signal corresponding to the first time period by a forward signal to obtain a first signal;

multiply an interference spectrum signal corresponding to the second time period by a reverse signal to obtain a second signal; and

obtain scale-transformed signals based on at least one first signal and at least one second signal.

In a possible implementation, the apparatus further includes a denoising module configured to denoise the axial frequency domain signals within the logarithmic space, to obtain denoised axial frequency domain signals, which is specifically configured to:

determine a segmentation threshold;

if an amplitude of an axial frequency domain signal is greater than or equal to the segmentation threshold, the denoised axial frequency domain signal is equal to an axial frequency domain signal in the logarithmic space; and

if the amplitude of the axial frequency domain signal is less than the segmentation threshold, the denosed axial frequency domain signal is equal to the segmentation threshold.

In a possible implementation, the denoising module determines the segmentation threshold, which is specifically configured to:

extract noise signals corresponding to all the axial frequency domain signals based on a clustering algorithm; and

determine the segmentation threshold based on the noise signals.

In a possible implementation, the decorrelation module 1004 performs decorrelation calculation on each set of single-scale signals to obtain single-scale blood flow signals, which is specifically configured to:

determine first-order statistics, second-order statistics, and third-order statistics of each set of single-scale signals; and
perform the decorrelation calculation based on the first-order statistics, the second-order statistics, and the third-order statistics to determine the single-scale blood flow signals.

In a possible implementation, the apparatus further includes an alignment module, and after performing Fourier transform on each scale-transformed signal and taking the logarithm, to obtain the axial frequency domain signal in the logarithmic space, the alignment module is specifically configured to:

perform an alignment operation on all the axial frequency domain signals based on a phase correlation algorithm;

where the denoised axial frequency domain signals are signals obtained by denoising the axial frequency domain signals after the alignment operation.

In a possible implementation, the apparatus further includes a dispersion compensation module which is specifically configured to:
prior to performing scale transform on the respective interference spectrum signals in the N interference spectrum signals based on the k scales, performing dispersion compensation on the respective interference spectrum signals in the time domain signals.

The apparatus for optical coherence tomography angiography provided by the embodiments of the present disclosure can be applied to the method embodiments as described above, and details are omitted here for brevity.

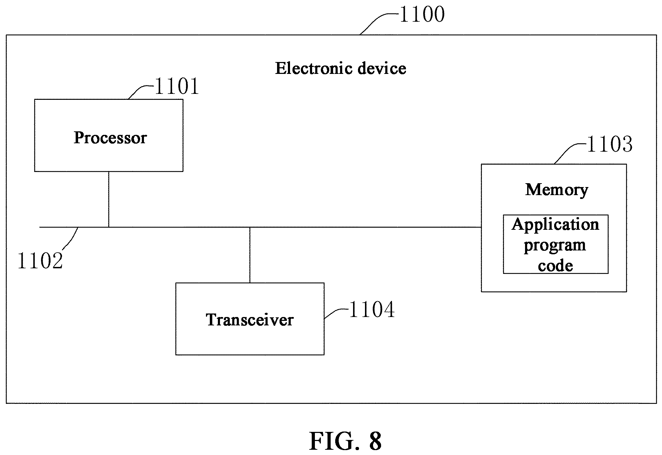

In the embodiments of the present disclosure, there is provided an electronic device. As shown in Fig. 8, the electronic device 1000 includes: a processor 1001 and a memory 1003. The processor 1001 and the memory 1003 are connected, for example, via a bus 1002. Alternatively, the electronic device 1000 may further include a transceiver 1004. It is worth noting that the amount of transceiver 1004 is not confined to one in actual application, and the structure of the electronic device 1000 does not formulate limitation to the embodiments of the present disclosure.

The processor 1001 may be a Central Processing unit (CPU), a general-purpose processor, a Digital Signal Processor (DSP), an Application Specific Integrated Circuit (ASIC), a Field Programmable Gate Array (FPGA) or other programmable logic logical device, a transistor logic device, a hardware component, or any combination thereof. It can be implemented or executed in combination with various example logic blocks, modules, and circuits as described here. The processor 1001 may be a combination for implementing computing functions, for example, a combination of one or more microprocessors, a combination of a DSP and a microprocessor, and the like.

The bus 1002 may include a path for communicating information among those components as described above. The bus 1002 may be a Peripheral Component Interconnect (PCI) bus, an Extended Industry Standard Architecture (EISA) bus, or the like. The bus 1002 can be divided into an address bus, a data bus, a control bus, and the like. For ease of description, it is only represented by a thick line in Fig. 8, which does not mean there is only one bus or one type of bus.

The memory 1003 may be a Read Only Memory (ROM) or other type of static storage device that can store static information and instructions, or a Random Access Memory (RAM) or other type of dynamic storage device that can store information and instructions, or may be an Electrically Erasable Programmable Read Only Memory (EEPROM), a Compact Disc Read Only Memory (CD-ROM) or other optical disc storage, optical disk storage (including a compressed optical disk, a laser disk, an optical disk, a digital versatile disk, a blue-ray disk, and the like), a magnetic disk storage medium or other magnetic storage device, or any other medium that can carry or store desired program code in the form of instructions or a data structure can be accessed by a computer, which is not limited to those listed above.

The memory 1003 is used to store application program code for executing solutions of the present disclosure, which is controlled and executed by the processor 1001. The processor 1001 is used to execute the application program code stored in the memory 1003, to implement the contents of the method embodiments as described above.

The electronic device includes, but is not limited to, a mobile terminal such as a mobile phone, a notebook computer, a digital broadcast receiver, a Digital Personal Assistant (PDA), a PAD (tablet computer), a Portable Multimedia Player (PMP), and the like, and a fixed terminal such as a digital TV, a desktop computer, and the like, or may be a server or the like. The electronic device as shown in Fig. 8 is only an example, which should not be construed as any limitation to the functions and application range of the embodiments of the present disclosure.

The embodiments of the present application provide a computer readable storage medium having a computer program stored thereon, which causes, when being operating on a computer, the computer to implement the corresponding contents of the method embodiments as describe above. As compared with the prior art, in the embodiments of the present disclosure, when a user reserves a certain service type for a certain time period, the fact whether source time periods related to an actual occupied time period in other types of services have been occupied is determined based on the actual occupied time period and a corresponding time period occupancy threshold, rather than directly being determined that the source time periods corresponding to other types of services have been occupied. In other words, if it is determined based on the actual occupied time period that a source time period of other type of service related thereto is available, which time can be reserved by a user. In this way, the present application can reduce time fragments and waste of resources, and further can reduce the waiting time for medical staffs and patients to improve the diagnosis efficiency.

It would be appreciated that, although various steps in the flowchart of the accompanying drawings are depicted sequentially following the indications of the arrows, those steps are not necessarily executed sequentially in the order indicated by the arrows. Unless specified otherwise, there is no strict order restriction on execution of those steps, and they can be executed in other orders. Moreover, at least some of the steps in the flowchart of the accompanying drawings may include multiple sub-steps or multiple stages. These sub-steps or stages are not necessarily executed at the same time but can be executed at different times, nor are they necessarily performed sequentially but can be performed in turn or alternately with at least some other steps or sub-steps or stages of other steps.

The above are only some implementations of the present disclose. It should be noted that, without departing from the principle of the present disclosure, the ordinary skilled in the art could also make some improvements and modifications which should also be considered as falling into the scope of protection of the present disclosure.

**Claims**

1. A method for optical coherence tomography angiography, comprising:

   acquiring time domain signals of a target area, the time domain signals being N interference spectrum signals obtained by repeating A-scans on the target area N times;
   performing scale transform on respective interference spectrum signals in the N interference spectrum signals based on k scales, to obtain N × k scale-transformed signals;
   wherein performing scale transform on any interference spectrum signal based on the k scales comprises: decomposing the any interference spectrum signal based on each of the k scales, to obtain k scale-transformed signals corresponding to the any interference spectrum signal;
   performing Fourier transform on each of the scale-transformed signals and taking a logarithm, to obtain an axial frequency domain signal in a logarithmic space, and denoising the axial frequency domain signal;
   dividing, based on the k scales, N × k of the axial frequency domain signals denoised into k sets of single-scale signals, and performing decorrelation calculation on each set of the single-scale signals to obtain single-scale blood flow signals;
   obtaining a multi-scale blood flow signal based on k sets of the single-scale blood flow signals; and
   obtaining a blood flow image based on the multi-scale blood flow signal.

2. The method of claim 1, **characterized in that** when the interference spectrum signals are corresponded to a sampling time period T, decomposing the any interference spectrum signals based on each of the k scales, to obtain the k scale-transformed signals corresponding to the any interference spectrum signal, comprises:

   decomposing the any interference spectrum signal based on any scale in the k scales, to obtain scale-transformed signals of the any scale corresponding to the any interference spectrum signal, comprising:

   determining a decomposed signal corresponding to the any scale, the decomposed signal being a signal within the sampling time period T, the decomposed signal comprising at least one time set that includes a first time period and a second time period, two of the first time periods adjacent to each other, or two of the second time periods adjacent to each other;
   multiplying the any interference spectrum signal corresponding to the first time period by a forward signal to obtain a first signal;
   multiplying the any interference spectrum signal corresponding to the second time period by a reverse signal to obtain a second signal; and
   obtaining, based on at least one of the first signal and at least one of the second signal, scale-transformed signals of the any interference spectrum signal corresponding to the any scale.

3. The method of claim 1, **characterized in that** denoising the axial frequency domain signal within the logarithmic space, to obtain the axial frequency domain signal denoised, comprises:

   determining a segmentation threshold;
   if an amplitude of the axial frequency domain signal is greater than or equal to the segmentation threshold, the axial frequency domain signal denoised being equal to the axial frequency domain signal within the logarithmic space; and
   if the amplitude of the axial frequency domain signal is less than the segmentation threshold, the axial frequency domain signal denoised being equal to the segmentation threshold.

4. The method of claim 3, **characterized in that** the determining the segmentation threshold comprises:

   extracting, based on a clustering algorithm, noise signals corresponding to all of the axial frequency domain signals; and
   determining the segmentation threshold based on the noise signals.

5. The method of claim 1, **characterized in that** the performing decorrelation calculation on each set of the single-scale signals to obtain the single-scale blood flow signals, comprising:

   determining first-order statistics, second-order statistics, and third-order statistics of each set of the single-scale signals; and
   performing the decorrelation calculation based on the first-order statistics, the second-order statistics, and the third-order statistics to determine the single-scale blood flow signals.

6. The method of claim 1, **characterized in that** after performing the Fourier transform on each of the scale-transformed signals and taking the logarithm, to obtain the axial frequency domain signal in the logarithmic space, it further comprises:
   performing, based on a phase correlation algorithm, an alignment operation on all of the axial frequency domain signals.

7. The method of claim 1, **characterized in that** prior to the performing scale transform on the respective interference spectrum signals in the N interference spectrum signals based on the k scales, it further comprises: performing dispersion compensation on the respective interference spectrum signals in the time domain signals.

8. An apparatus for optical coherence tomography angiography, comprising:

   an acquiring module configured to acquire time domain signals of a target area, the time domain signals being N interference spectrum signals obtained by repeating A-scans on the target area N times;
   a scale-transforming module configured to perform scale transform on respective interference spectrum signals in the N interference spectrum signals based on k scales, to obtain N × k scale-transformed signals;
   wherein performing scale transform on any interference spectrum signal based on the k scales comprises: decomposing the any interference spectrum signal based on each of the k scales, to obtain k scale-transformed signals corresponding to the any interference spectrum signal;
   a frequency domain transforming module configured to perform Fourier transform on each of the scale-transformed signals and take a logarithm, to obtain an axial frequency domain signal in a logarithmic space, and denoise the axial frequency domain signal;
   a decorrelation module configured to divide, based on the k scales, N × k of the axial frequency domain signals denoised into k sets of single-scale signals, and perform decorrelation calculation on each set of the single-scale signals to obtain single-scale blood flow signals;
   a fusion module configured to obtain a multi-scale blood flow signal based on k sets of the single-scale blood flow signals; and
   an imaging module configured to obtain a blood flow image based on the multi-scale blood flow signal.

9. An electronic device, **characterized in that** it comprises:

   one or more processors; and
   a memory;
   wherein one or more computer programs are stored in the memory and configured to be executed by the one or more processors, and the one or more computer programs are configured to execute the method for optical coherence tomography angiography of any one of claims 1-7.

10. A computer readable storage medium, **characterized in that** it comprises a computer program that can be loaded by a processor and configured to execute the method for optical coherence tomography angiography of any one of claims 1-7.

Acquiring time domain signals of a target area — S101

Performing scale transform on respective interference spectrum signals in the N interference spectrum signals based on k scales, to obtain N × k scale-transformed signals — S102

Performing Fourier transform on each of the scale-transformed signals and taking a logarithm, to obtain an axial frequency domain signal in a logarithmic space — S103

Dividing, based on the k scales, N × k of the axial frequency domain signals denoised into k sets of single-scale signals, and performing decorrelation calculation on each set of the single-scale signals to obtain single-scale blood flow signals — S104

Obtaining multi-scale blood flow signals based on k sets of the single-scale blood flow signals — S105

Obtaining a blood flow image based on the multi-scale blood flow signal — S106

FIG. 1

FIG. 2

（a）

（b）

（c）

FIG. 3

S102

| Determining a decomposed signal corresponding to the any scale | S021 |

| Multiplying the any interference spectrum signal corresponding to the first time period by a forward signal to obtain a first signal; and multiplying the any interference spectrum signal corresponding to the second time period by a reverse signal to obtain a second signal | S022 |

| Obtaining scale-transformed signals based on at least one first signal and at least one second signal | S023 |

FIG. 4

Determining a segmentation threshold — S031

If an amplitude of the axial frequency domain signal is greater than or equal to the segmentation threshold, the axial frequency domain signal denoised being equal to the axial frequency domain signal within the logarithmic space — S032

If the amplitude of the axial frequency domain signal is less than the segmentation threshold, the axial frequency domain signal denoised being equal to the segmentation threshold — S033

FIG. 5

FIG. 6

FIG. 7

FIG. 8

## INTERNATIONAL SEARCH REPORT

| International application No. |
| --- |
| **PCT/CN2022/120404** |

**A.    CLASSIFICATION OF SUBJECT MATTER**

G06T 11/00(2006.01)i

According to International Patent Classification (IPC) or to both national classification and IPC

**B.    FIELDS SEARCHED**

Minimum documentation searched (classification system followed by classification symbols)

G06T; G06K; G06V; A61B

Documentation searched other than minimum documentation to the extent that such documents are included in the fields searched

Electronic data base consulted during the international search (name of data base and, where practicable, search terms used)

CNABS; CNTXT; VEN; USTXT; WOTXT; EPTXT; CNKI; 百度, BAIDU; IEEE: 光学相干层析, 光学相干断层, 血管造影, 血流, 扫描, 尺度, 变换, 分段, 傅里叶, 傅立叶, 对数, 去噪, 去相关, optical coherence tomography, OCT, angiography, OCTA, blood flow, scan, scale, transform, segment, fourier, logarithm, denoise, decorrelation

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 114037769 A (TOWARDPI (BEIJING) MEDICAL TECHNOLOGY CO., LTD.) 11 February 2022 (2022-02-11) description, paragraphs 2-122 | 1-10 |
| Y | CN 108245130 A (NANJING UNIVERSITY OF SCIENCE AND TECHNOLOGY) 06 July 2018 (2018-07-06) description, paragraphs 2-43 | 1, 3-10 |
| Y | CN 110101362 A (CIXI INSTITUTE OF BIOMEDICAL ENGINEERING, NINGBO INSTITUTE OF INDUSTRIAL TECHNOLOGY, CHINESE ACADEMY OF SCIENCES et al.) 09 August 2019 (2019-08-09) description, paragraphs [0002]-[0048] | 1, 3-10 |
| Y | CN 111436905 A (BEIJING TOPI IMAGING TECHNOLOGY CO., LTD.) 24 July 2020 (2020-07-24) description, paragraphs 54-123 | 1, 3-10 |
| A | CN 109907731 A (ZHEJIANG UNIVERSITY) 21 June 2019 (2019-06-21) entire document | 1-10 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **06 December 2022** | **14 December 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/120404** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2021267457 A1 (NEW JERSEY INSTITUTE OF TECHNOLOGY) 02 September 2021 (2021-09-02) <br> entire document | 1-10 |

Form PCT/ISA/210 (second sheet) (January 2015)

## INTERNATIONAL SEARCH REPORT
### Information on patent family members

International application No.

**PCT/CN2022/120404**

| Patent document cited in search report | | | Publication date (day/month/year) | Patent family member(s) | | | Publication date (day/month/year) |
|---|---|---|---|---|---|---|---|
| CN | 114037769 | A | 11 February 2022 | None | | | |
| CN | 108245130 | A | 06 July 2018 | CN | 108245130 | B | 04 June 2021 |
| CN | 110101362 | A | 09 August 2019 | CN | 110101362 | B | 17 December 2021 |
| CN | 111436905 | A | 24 July 2020 | None | | | |
| CN | 109907731 | A | 21 June 2019 | US | 2022248960 | A1 | 11 August 2022 |
| | | | | WO | 2020155415 | A1 | 06 August 2020 |
| | | | | CN | 109907731 | B | 01 June 2021 |
| US | 2021267457 | A1 | 02 September 2021 | None | | | |

Form PCT/ISA/210 (patent family annex) (January 2015)